(19) Europäisches Patentamt
European Patent Office
Office européen des brevets

(11) Numéro de publication : **0 511 074 A1**

(12) **DEMANDE DE BREVET EUROPEEN**

(21) Numéro de dépôt : **92401110.9**

(22) Date de dépôt : **21.04.92**

(51) Int. Cl.[5] : **C07D 275/06,** C07D 221/12, C07D 235/26, C07D 417/06, C07D 401/06, A61K 31/425, A61K 31/435, A61K 31/415

(30) Priorité : **26.04.91 FR 9105169**

(43) Date de publication de la demande : **28.10.92 Bulletin 92/44**

(84) Etats contractants désignés : **PT**

(71) Demandeur : **RHONE-POULENC RORER SA
20, avenue Raymond Aron
F-92160 Antony (FR)**

(72) Inventeur : **Damour, Dominique
18 rue Henri Ragnault
F-75014 Paris (FR)**
Inventeur : **Labaudinière, Richard
1 place de Valois
F-94220 Charenton (FR)**
Inventeur : **Malleron, Jean-Luc
2 allée Renoir
F-91460 Marcoussis (FR)**
Inventeur : **Mignani, Serge
129 avenue du général Leclerc
F-75014 Paris (FR)**

(74) Mandataire : **Savina, Jacques et al
Rhône-Poulenc Rorer S.A. Direction Brevets
(t144) 20 avenue Raymond Aron
F-92165 Antony Cédex (FR)**

(54) **Dérivés hétérocycliques antisérotonines, leur préparation et les médicaments les contenant.**

(57) Composés de formule :

$$R_1\text{-}(CH_2)_n\text{-Het} \qquad (I)$$

dans laquelle
— $R_1$ représente un reste de formule :

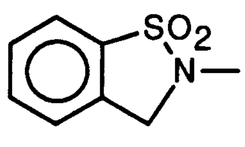

(A)

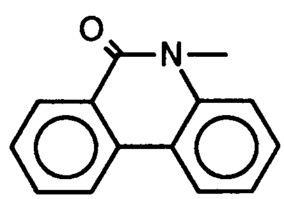

(B)

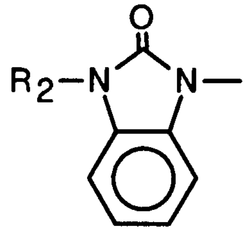

(C)

Net représente :
. un radical phényl-4 tétrahydro-1,2,3,6 pyridyl-1 dont le cycle phényle est éventuellement substitué par un atome d'halogène ou un radical alkyle, alcoxy ou hydroxy,
. un radical phényl-4 pipéridino dont le cycle phényle est éventuellement substitué par un atome d'halogène ou un radical alkyle, alcoxy ou hydroxy,

EP 0 511 074 A1

. un radical phényl-4 pipérazinyl-1 dont le cycle phényle est éventuellement substitué par un atome d'halogène ou un radical alkyle, alcoxy ou hydroxy,

— $R_2$ représente un atome d'hydrogène ou un radical alkyle,

— n est égal à 1, 2, 3 ou 4,

leurs sels, leur préparation et les médicaments les contenant.

La présente invention concerne des composés de formule:

$R_1-(CH_2)_n-Het$ (I)

leur préparation et les médicaments les contenant.

Dans la formule (I),

- $R_1$ représente un reste de formule:

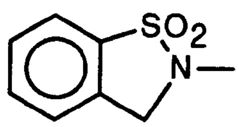
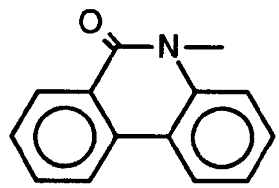

(A) (B)

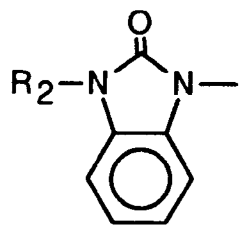

(C)

- Het représente

un radical phényl-4 tétrahydro-1,2,3,6 pyridyl-1 dont le cycle phényle est éventuellement substitué par un atome d'halogène ou un radical alkyle, alcoxy ou hydroxy,

un radical phényl-4 pipéridino dont le cycle phényle est éventuellement substitué par un atome d'halogène ou un radical alkyle, alcoxy ou hydroxy,

un radical phényl-4 pipérazinyl-1 dont le cycle phényle est éventuellement substitué par un atome d'halogène ou un radical alkyle, alcoxy ou hydroxy,

- $R_2$ représente un atome d'hydrogène ou radical alkyle,
- n est égal à 1, 2, 3 ou 4.

L'invention concerne également les sels des composés de formule (I) avec les acides minéraux ou organiques.

Dans les définitions qui précédent et celles qui seront citées ci-après, les radicaux alkyle et alcoxy contiennent 1 à 4 atomes de carbone en chaîne droite ou ramifiée et les atomes d'halogène sont de préférence les atomes de fluor, de chlore ou de brome.

Les composés de formule (I) à l'exception de ceux pour lesquels $R_1$ représente un reste de formule (C) dans laquelle $R_2$ représente un atome d'hydrogène peuvent être préparés par action d'un dérivé de formule :

$R_1H$ (II)

dans laquelle $R_1$ a les mêmes significations que ci-dessus sur un dérivé halogéné de formule:

$Hal-(CH_2)_n-Het$ (III)

dans laquelle Hal représente un atome d'halogène , Het et n ont les mêmes significations que dans la formule (I).

Cette réaction s'effectue généralement en présence d'une base telle qu'un hydrure de métal alcalin, un hydroxyde de métal alcalin, un bicarbonate de métal alcalin ou un carbonate de métal alcalin, au sein d'un solvant inerte tel que le diméthylformamide ou le tétrahydrofuranne, à une température comprise entre 20°C et la température d'ébullition du solvant.

Les composés de formule (II) sont commercialisés ou peuvent être obtenus par application ou adaptation des méthodes décrites par B. L. HAWBECKER, J. Chem. Educ., 53 (6), 398 (1976) et dans le brevet US 3164602.

Les dérivés halogénés de formule (III) peuvent être obtenus par action d'une amine de formule :

Het-H (IV)

dans laquelle Het a les mêmes significations que dans la formule (I) sur un dérivé dihalogéné de formule :

$Hal-(CH_2)_n-Hal'$ (V)

dans laquelle Hal et Hal' représentent un atome d'halogène, les deux atomes d'halogène pouvant être identiques ou différents et n a les mêmes significations que dans la formule (I).

Cette réaction s'effectue généralement au sein d'un solvant inerte tel que le diméthylformamide ou l'acétonitrile, en présence d'une base telle qu'un carbonate de métal alcalin, à une température comprise entre 20°C et la température d'ébullition du solvant.

Les amines de formule (IV) sont commercialisées ou peuvent être obtenues par application ou adaption des méthodes décrites par D. K. YUNK et coll., J. Med. Chem., 21, 1301 (1978); L. THUNUS et coll., Ann. Pharm., 38, 353 (1980); L. GOOTES et coll., Arzneim. Forsch., 17, 1145 (1963) et dans le brevet EP 350403.

Les composés de formule (I) à l'exception de ceux pour lesquels $R_1$ représente un reste de formule (C) dans laquelle $R_2$ représente un atome d'hydrogène peuvent également être préparés par action d'un dérivé de formule :

$$R_1\text{-}(CH_2)_n\text{-Hal} \qquad \text{(VI)}$$

dans laquelle $R_1$ a les mêmes significations que ci-dessus, n a les mêmes significations que dans la formule (I) et Hal représente un atome d'halogène, sur une amine de formule (IV).

Cette réaction s'effectue généralement au sein d'un solvant inerte tel que le diméthylformamide ou le tétrahydrofuranne, en présence d'une base telle qu'un bicarbonate de métal alcalin ou une trialkylamine, à la température d'ébullition du solvant.

Les dérivés de formule (VI) peuvent être obtenus par action d'un dérivé de formule (II) sur un dérivé dihalogéné de formule (V).

Cette réaction s'effectue dans un solvant inerte tel que le diméthylformamide, au moyen d'hydrure de sodium, à une température comprise entre 20°C et la température d'ébullition du solvant.

Les composés de formule (I) pour lesquels $R_1$ représente un reste de formule (C) dans laquelle $R_2$ représente un atome d'hydrogène peuvent être préparés par hydrolyse d'un dérivé de formule:

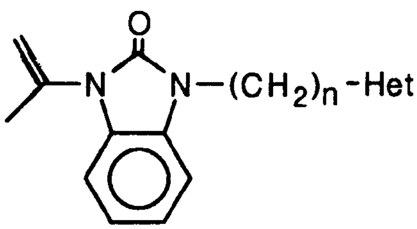

(VII)

dans laquelle Het et n ont les mêmes significations que dans la formule (I).

Cette hydrolyse s'effectue en milieu acide (acide sulfurique, acide chlorhydrique par exemple), au sein d'un solvant inerte tel qu'un alcool (méthanol, éthanol par exemple), à une température comprise entre 20°C et la température d'ébullition du solvant.

Les composés de formule (VII) peuvent être obtenus par application ou adaptation de la méthode décrite dans les exemples.

Les composés de formule (I) pour lesquels $R_1$ représente un reste de formule (C) dans laquelle $R_2$ représente un radical alkyle peuvent être obtenus par alkylation des composés correspondants pour lesquels $R_2$ représente un atome d'hydrogène.

Cette réaction s'effectue généralement au moyen d'un halogènure d'alkyle (iodure de méthyle, bromure d'éthyle par exemple), au sein d'un solvant inerte tel que le tétrahydrofuranne, en présence d'une base telle que le triméthylsilanonate de potassium, à une température voisine de 20°C.

Les mélanges réactionnels obtenus par les divers procédés décrits précédemment sont traités suivant des méthodes classiques physiques (extraction, évaporation, distillation, chromatographie ...) ou chimiques (formation de sels ...).

Les composés de formule (I) sous forme de base libre peuvent éventuellement être transformés en sels d'addition avec un acide minéral ou organique, par action d'un tel acide au sein d'un solvant organique tel qu'un alcool, une cétone, un éther ou un solvant chloré.

Les composés de formule (I) et leurs sels présentent des propriétés intéressantes. Ces composés possèdent des propriétés antagonistes de la sérotonine (récepteurs 5HT2) et sont donc utiles pour le traitement des affections où la sérotonine est impliquée, notamment les affections du système nerveux central, du système cardiovasculaire et les troubles gastrointestinaux.

Ces composés sont , en particulier, utiles pour le traitement de l'anxiété, des troubles du sommeil, des psychoses et notamment de la schizophrénie, de la migraine, de l'asthme, de l'hypertension et de l'urticaire, comme analgésiques et comme inhibiteurs de l'aggrégation plaquettaire.

L'affinité des composés de formule (I) pour les sites récepteurs centraux à sérotonine (type S2) a été déterminée selon une technique inspirée de celle de J. E. LEYSEN et coll., Mol. Pharmacol., 21, 301 (1982) qui consiste à mesurer l'affinité des produits pour les sites de liaison de la kétansérine tritiée. Dans ce test, la $CI_{50}$ des composés de formule (I) est généralement inférieure à 25 nM.

Les composés de formule (I) présentent un toxicité faible. Ils sont généralement atoxiques à 300 mg/kg par voie orale chez la souris en administration unique.

Pour l'emploi thérapeutique, il peut être fait usage des composés de formule (I) tels quels ou à l'état de sels pharmaceutiquement acceptables.

Comme sels pharmaceutiquement acceptables, peuvent être cités les sels d'addition avec les acides minéraux tels que chlorhydrates, sulfates, nitrates, phosphates ou organiques tels que acétates, propionates, succinates, oxalates, benzoates, fumarates, maléates, méthanesulfonates, iséthionate, théophillineacétates, phénolphtalinates, salicylates, méthylène-bis-ß oxynaphtoates ou des dérivés substitués de ces composés.

Les exemples suivants donnés à titre non limitatif montrent comment l'invention peut être mise en pratique.

**EXEMPLE 1**

1,69 g de 2H-benzisothiazoline dioxyde-1,1, 2,56 g de (chloro-3 propyl)-1 (fluoro-4 phényl)-4 pipérazine et 0,84 g de bicarbonate de sodium dans 25 cm3 de N,N-diméthylformamide sec sont agités au reflux du solvant pendant 5 heures. Le mélange réactionnel est refroidi et versé dans un mélange de 50 cm3 d'eau et 50 cm3 de dichlorométhane. La phase organique est décantée, lavée à l'eau (4 fois 30 cm3), séchée sur sulfate de magnésium et concentrée à sec sous pression réduite (2,4 kPa). Le résidu est chromatographié sur une colonne de gel de silice sous une pression de 0,7 bar par du dichlorométhane puis par de l'acétate d'éthyle. Le solide obtenu est recristallisé dans 8 cm3 d'acétonitrile bouillant. On obtient 0,43 g de [[(fluoro-4 phényl)-4 pipérazinyl-1]-3 propyl]-2 2H-benzisothiazoline dioxyde- 1,1 fondant à 116°C.

La (chloro-3 propyl)-1 (fluoro-4 phényl)-4 pipérazine peut être préparée de la manière suivante : une solution de 68 cm3 de bromo-1 chloro-3 propane et 50 g de (fluoro-4 phényl)-4 pipérazine dans 400 cm3 d'acétonitrile est agitée 20 heures à 25°C avec 97 g de carbonate de potassium. Le mélange est filtré puis concentré à sec sous pression réduite (2,7 kPa). Le résidu est chromatographié sur une colonne de gel de silice (0,2-0,083 mm, diamètre 9 cm, hauteur 60 cm) en éluant par de l'acétate d'éthyle et en recueillant des fractions de 500 cm3. Les fractions 5 à 7 sont réunies et concentrées à sec sous pression réduite (2,7 kPa) pour donner 44,4 g de (chloro-3 propyl)-1 (fluoro-4 phényl)-4 pipérazine sous la forme d'une huile utilisée telle quelle dans les synthèses ultérieures.

Le 2H-benzisothiazoline dioxyde-1,1 peut être préparé selon la méthode décrite dans le brevet US 3164602.

**Exemple 2**

On mélange 1,95 g de phénanthridone, 0,1 g de bromure de tétrabutyl ammonium, 4,5 g de carbonate de potassium, 2,6 g de (chloro-3 propyl)-1 (fluoro-4 phényl)-4 pipérazine dans 70 cm3 de diméthylformamide. Le mélange est chauffé 5 heures à ébullition puis refroidi à une température voisine de 20°C. Le filtrat est évaporé à sec à 40°C sous pression réduite (10 mm de mercure; 1,35 kPa). Le résidu est repris par 20 cm3 d'eau et extrait par trois fois 50 cm3 d'acétate d'éthyle. Les phases organiques sont rassemblées, séchées sur sulfate de magnésium anhydre et évaporées à sec à 40°C sous pression réduite (20 mm de mercure; 2,7 kPa). L'huile obtenue est purifiée par flash-chromatographie sur colonne de silice, sous courant d'azote à moyenne pression (0,1-1,5 bar) avec un mélange dichlorométhane-méthanol (99-1 en volumes) comme éluant. On obtient 1,7 g d'un solide jaune qui recristallisé dans 40 cm3 d'éthanol chaud conduit à 1,3 g de {[(fluoro-4 phényl)-4 pipérazinyl-1]-3 propyl}-1 phénanthridone fondant à 140°C.

La phénanthridone peut être préparée selon la méthode décrite par B. L. HAWBECKER, J. Chem. Educ., 53(6), 398 (1976).

**EXEMPLE 3**

A une solution de 2,1 g de [[(fluoro-4 phényl)-4 pipérazinyl-1]-3 propyl]-1 isopropényl-3 2H-benzimidazolinone-2 dans 30 cm3 d'éthanol, on coule 2,65 cm3 d'acide chlorhydrique 6N à une température voisine de 20°C. La solution est chauffée 2 heures et 30 minutes à ébullition puis refroidie à une température proche de 20°C. Le milieu réactionnel est repris par 30 cm3 d'eau distillée, 30 cm3 de dichlorométhane et alcalinisée à pH 9 par de la soude 5N. La phase organique est extraite par 4 fois 30 cm3 de dichlorométhane, séchée sur sulfate de magnésium, traitée au noir végétal et concentrée à sec à 40°C sous pression réduite (2,7 kPa). Le

résidu est recristallisé dans 10 cm3 d'acétonitrile bouillant. On obtient ainsi 1 g de [[(fluoro-4 phényl)-4 pipérazinyl-1]-3 propyl]-1 2H-benzimidazolinone-2 fondant à 145°C.

La [[(fluoro-4 phényl)-4 pipérazinyl-1]-3 propyl]-1 isopropényl-3 2H-benzimidazolinone-2 peut être préparée de la manière suivante : 1,74 g d'isopropényl-3 2H-benzimidazolinone-2, 2,56 g de (chloro-3 propyl)-1 (fluoro-4 phényl)-4 pipérazine et 0,84 g de bicarbonate de sodium dans 20 cm3 de N,N-diméthylformamide sec sont agités au reflux du solvant pendant 6 heures. Le mélange réactionnel est refroidi et versé dans un mélange de 30 cm3 d'eau et 30 cm3 de dichlorométhane. La phase organique est décantée, lavée à l'eau (3 fois 30 cm3), séchée sur sulfate de magnésium et concentrée à sec sous pression réduite (2,7 kPa). Le résidu est chromatographié sur une colonne de gel de silice sous une pression de 0,7 bar en éluant avec un mélange dichlorométhane-acétate d'éthyle (50-50 en volumes). On obtient ainsi 2,1 g de [[(fluoro-4 phényl)-4 pipérazinyl-1]-3 propyl]-1 isopropényl-3 2H-benzimidazolinone-2 sous forme d'une huile utilisée telle quelle dans les synthèses ultérieures.

L'isopropényl-3 2H-benzimidazolinone-2 peut être préparée selon la méthode décrite par O. METH-COHN, J. Chem. Soc., PT1, 261 (1982).

**EXEMPLE 4**

A une solution de 4,1 g de [[(fluoro-4 phényl)-4 pipérazinyl-1]-3 propyl]-1 2H-benzimidazolinone-2 dans 100 cm3 de tétrahydrofuranne on ajoute par portions 1,83 g de triméthylsilanonate de potassium, à une température proche de 20°C. L'agitation est maintenue 30 minutes puis on coule goutte à goutte 0,81 cm3 d'iodure de méthyle. Le milieu réactionnel est agité 10 heures à une température proche de 20°C puis le précipité formé est filtré. Le filtrat est concentré à sec à 40°C sous pression réduite (2,7 kPa). Le résidu est recristallisé dans 10 cm3 d'acétonitrile bouillant et traité au noir végétal. On obtient ainsi 1,6 g de [[(fluoro-4 phényl)-4 pipérazinyl-1]-3 propyl]-1 méthyl-3 2H-benzimidazolinone-2 fondant à 105°C.

Les médicaments selon l'invention sont constitués par un composé de formule (I) sous forme libre ou sous forme d'un sel d'addition avec un acide pharmaceutiquement acceptable, à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être des solutions aqueuses ou non aqueuses, des suspensions ou des émulsions. Comme solvant ou véhicule on peut employer l'eau, le propylèneglycol, un polyéthylèneglycol, des huiles végétales, en particulier l'huile d'olive, des esters organiques injectables, par exemple l'oléate d'éthyle ou autres solvants organ iques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, pommades, lotions, collyres, collutoires, gouttes nasales ou aérosols.

En thérapeutique humaine, les composés selon l'invention sont particulièrement utiles pour le traitement des affections ou la sérotonine est impliquée et notamment les affections du système nerveux central, du système cardiovasculaire et les troubles intestinaux. Ils sont en particulier utiles pour le traitement de l'anxiété, des troubles du sommeil, de la dépression, des psychoses et notamment de la schizophrénie, de la migraine,

de l'asthme, de l'hypertension et de l'urticaire, comme analgésiques et comme inhibiteurs de l'agrégation plaquettaire.

Les doses dépendent de l'effet recherché et de la voie d'administration utilisée; elles sont généralement comprises entre 10 et 300 mg par jour par voie orale pour un adulte avec des doses unitaires allant de 5 à 150 mg de substance active.

D'une façon générale, le médecin déterminera la posologie appropriée en fonction de l'âge, du poids et de tous les autres facteurs propres au sujet à traiter.

Les exemples suivants illustrent des compositions selon l'invention:

EXEMPLE A

On prépare, selon la technique habituelle, des gélules dosées à 50 mg de produit actif ayant la composition suivante :

| | |
|---|---|
| - [[(fluoro-4 phényl)-4 pipérazinyl-1]-3 propyl]-1 2H-benzimidazolinone-2 | 50 mg |
| - Cellulose | 18 mg |
| - Lactose | 55 mg |
| - Silice colloïdale | 1 mg |
| - Carboxyméthylamidon sodique | 10 mg |
| - Talc | 10 mg |
| - Stéarate de magnésium | 1 mg |

EXEMPLE B

On prépare selon la technique habituelle de comprimés dosés à 50 mg de produit actif ayant la composition suivante :

| | |
|---|---|
| - [[(fluoro-4 phényl)-4 pipérazinyl-1]-3 propyl]-2 2H-benzisothiazoline dioxyde-1,1 | 50 mg |
| -Lactose | 104 mg |
| - Cellulose | 40 mg |
| - Polyvidone | 10 mg |
| - Carboxyméthylamidon sodique | 22 mg |
| - Talc | 10 mg |
| - Stéarate de magnésium | 2 mg |
| - Silice colloïdale | 2 mg |
| - Mélange d'hydroxyméthylcellulose, glycérine, oxyde de titane (72-3,5-24,5) q.s.p. 1 comprimé pelliculé terminé à | 245 mg |

EXEMPLE C

On prépare une solution injectable contenant 10 mg de produit actif ayant la composition suivante :

| | | |
|---|---|---|
| - [[(fluoro-4 phényl)-4 pipérazinyl-1]-3 propyl]-1 phénanthridone | | 10 mg |
| - Acide benzoïque | | 80 mg |
| - Alcool benzylique | | 0,06 cm3 |
| - Benzoate de sodium | | 80 mg |
| - Ethanol à 95 % | | 0,4 cm3 |
| - Hydroxyde de sodium | | 24 mg |
| - Propylène glycol | | 1,6 cm3 |
| - Eau | q.s.p. | 4 cm3 |

**Revendications**

**1 -** Composés de formule:

$$R_1\text{-}(CH_2)_n\text{-Het} \qquad (I)$$

dans laquelle
-$R_1$ représente un reste de formule:

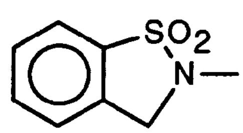

(A)

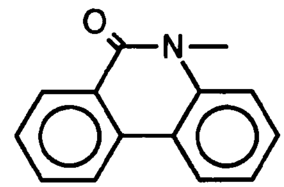

(B)

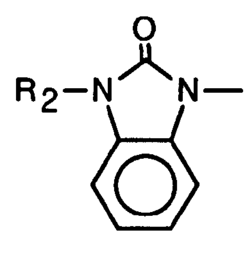

(C)

Het représente:

. un radical phényl-4 tétrahydro-1,2,3,6 pyridyl-1 dont le cycle phényle est éventuellement substitué par un atome d'halogène ou un radical alkyle, alcoxy ou hydroxy,

. un radical phényl-4 pipéridino dont le cycle phényle est éventuellement substitué par un atome d'halogène ou un radical alkyle, alcoxy ou hydroxy,

. un radical phényl-4 pipérazinyl-1 dont le cycle phényle est éventuellement substitué par un atome d'halogène ou un radical alkyle, alcoxy ou hydroxy,

-$R_2$ représente un atome d'hydrogène ou un radical alkyle,
-n est égal à 1, 2, 3 ou 4
étant entendu que les radicaux alkyle et alcoxy contiennent 1 à 4 atomes de carbone en chaîne droite ou ramifiée, ainsi que les sels de ces composés avec un acide organique ou minéral.

**2 -** Procédé de préparation des composés de formule (I) selon la revendication 1 à l'exception de ceux pour lesquels $R_1$ représente un reste de formule (C) dans laquelle $R_2$ représente un atome d'hydrogène caractérisé en ce que l'on fait réagir un dérivé de formule :

$$R_1 H \qquad (II)$$

dans laquelle $R_1$ a les mêmes significations que ci-dessus sur un dérivé halogéné de formule:

$$Hal\text{-}(CH_2)_n\text{-}Het \qquad (III)$$

dans laquelle Hal représente un atome d'halogène, Het et n ont les mêmes significations que dans la revendication 1, isole le produit et le transforme éventuellement en sel.

**3 -** Procédé de préparation des composés de formule (I) selon la revendication 1 à l'exception de ceux pour lesquels $R_1$ représente un reste de formule (C) dans laquelle $R_2$ représente un atome d'hydrogène caractérisé en ce que l'on fait réagir un dérivé de formule :

$$R_1\text{-}(CH_2)_n\text{-}Hal \qquad (VI)$$

dans laquelle $R_1$ et n ont les mêmes significations que ci-dessus et Hal représente un atome d'halogène sur une amine de formule:

$$Het\text{-}H \qquad (IV)$$

dans laquelle Het a les mêmes significations que dans la revendication 1, isole le produit et le transforme éventuellement en sel.

**4 -** Procédé de préparation des composés de formule (I) pour lesquels $R_1$ représente un reste de formule (C) dans laquelle $R_2$ représente un atome d'hydrogène caractérisé en ce que l'on hydrolyse un dérivé de formule :

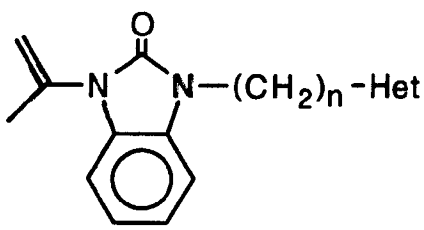

(VII)

dans laquelle n et Het ont les mêmes significations que dans la revendication 1, isole le produit et le transforme éventuellement en sel.

**5 -** Procédé de préparation des composés de formule (I) selon la revendication 1 pour lesquels $R_1$ représente un reste de formule (C) dans laquelle $R_2$ représente un radical alkyle caractérisé en ce que l'on alkyle un composé correspondant pour lequel $R_2$ représente un atome d'hydrogène, isole le produit et le transforme éventuellement en sel.

**6 -** Médicaments caractériés en ce qu'ils contiennent comme matière active au moins un composé de formule (I) selon la revendication 1.

**7 -** Médicaments selon la revendication 6 pour le traitement des maladies où la sérotonine est impliquée.

Office européen des brevets

# RAPPORT DE RECHERCHE EUROPEENNE

Numero de la demande

EP 92 40 1110

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| A | EP-A-0 350 403 (RHONE-POULENC SANTE)<br>* revendications * | 1,6,7 | C07D275/06<br>C07D221/12<br>C07D235/26<br>C07D417/06<br>C07D401/06<br>A61K31/425<br>A61K31/435<br>A61K31/415 |
| A | US-A-4 110 449 (PETER C. WADE)<br>* revendications * | 1,6,7 | |
| A | EP-A-0 332 528 (RHONE-POULENC SANTE)<br>* page 6, ligne 14 - page 6, ligne 28; revendications * | 1,6,7 | |
| A | US-A-4 250 176 (JAN VANDENBERK)<br>* revendications * | 1,6,7 | |
| A | US-A-4 254 127 (JAN VANDENBERK)<br>* colonne 11, ligne 25 - colonne 11, ligne 45; revendications * | 1,6,7 | |
| P,X | EP-A-0 429 341 (RHONE-POULENC SANTE)<br>* page 5, ligne 48 - page 6, ligne 21; revendications * | 1-7 | |
| | | | **DOMAINES TECHNIQUES RECHERCHES (Int. Cl.5 )** |
| | | | C07D |

Le présent rapport a été établi pour toutes les revendications

| Lieu de la recherche | Date d'achèvement de la recherche | Examinateur |
|---|---|---|
| LA HAYE | 26 JUIN 1992 | HENRY J.C. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)